# EUROPEAN PATENT APPLICATION

(11) **EP 4 524 531 A1**
(43) Date of publication of application: **19.03.2025**
(21) Application number: 22941602.9
(22) Date of filing: 10.05.2022
(51) Int. Cl.: G01K 11/12

(54) **TEMPERATURE CONTINUANCE DETECTOR AND HEATSTROKE DETECTOR**

(71) Applicant: Biodata Bank, Inc., Tokyo 150-0031 (JP)
(72) Inventor: SHIOTSU, Takahiro, Tokyo 1500031 (JP); OURA, Issei, Tokyo 1500031 (JP); KODERA, Yuya, Tokyo 1500031 (JP)
(74) Representative: Hasegawa, Kan
(86) International application number: PCT/JP2022/019784
(87) International publication number: WO 2023/218520

(57) **Abstract**

[Technical Problem] To provide a temperature continuity detector capable of accurately detecting that a detection object has maintained a state of being at a predetermined temperature or at a temperature equal to or higher than the predetermined temperature for a continued period.

[Solution] The present invention relates to a temperature continuity detector that detects at least one of the following: that a detection object generating heat has maintained a predetermined temperature for a predetermined time, or that a temperature equal to or higher than the predetermined temperature has continued for a predetermined time. The temperature continuity detector comprises a sensor unit having a temperature-sensitive material that produces a specific change in response to the predetermined temperature and a heat conductive material that conducts heat generated by the detection object to the temperature-sensitive material over the predetermined time.

## Description

### [Technical Field]

This invention relates to temperature continuity detectors and heat stroke detectors.

### [Background Art]

A heat stroke warning sticker has been proposed in which a portion of the material becomes transparent when the temperature exceeds a predetermined temperature, making the underlying "warning label" visible (see Patent Document 1). These constitute a part of this document by reference.

### [Citation List]

### [Patent Literature]

[PTL 1] Japanese Patent Application Publication No. 2018-179685.

### [Summary of Invention]

### [Technical Problem]

In general, regarding the mechanism of heat stroke, it is important to detect that the core body temperature has become equal to or higher than a predetermined temperature. Therefore, heat stroke cannot be immediately judged just because the surface temperature has become equal to or higher than a predetermined temperature temporarily or instantaneously. Therefore, the heat stroke warning sticker described in PTL 1 is insufficient as a method for making an accurate judgment of whether or not it is heat stroke.

In addition, the heat stroke warning sticker described in Patent Literature 1 also produces a "warning indication" when the temperature momentarily exceeds the detection temperature due to temporary and incidental factors (e.g., temporarily being near a heat source, temporarily entering or passing through a high-temperature space). Furthermore, there is no way to verify whether this "warning indication" is a false detection. For example, even if a person judges it to be a temporary false detection when the "warning indication" appears due to being temporarily near a heat source, this judgment could be dangerous if the person was already in a state of progressing heat stroke, as there would be a discrepancy between the person's self-awareness and their actual physiological condition.

Thus, not only is it difficult to make accurate judgments due to the nature of the product, but it is also problematic from a safety perspective that when false detection occurs, it is difficult to verify such false detection.

Therefore, one object of the present invention is to solve these problems.

### [Solution to Problem]

According to the present invention, there is provided:
A temperature continuity detector that detects at least one of the following: that the detected object that generates heat has maintained a predetermined temperature for a predetermined time, or that a temperature equal to or higher than the predetermined temperature has continued for a predetermined time,
wherein the temperature continuity detector comprises a sensor unit having a temperature-sensitive material that produces a specific change in response to the predetermined temperature, and a heat conductive material that conducts the heat generated by the detected object to the temperature-sensitive material over the predetermined time.

Also, according to the present invention, there is provided:
A heatstroke detector that detects at least one of the following: that a living body has maintained a predetermined temperature for a predetermined time, or that a temperature equal to or higher than the predetermined temperature has continued for a predetermined time,
wherein the heatstroke detector comprises a sensor unit having a temperature-sensitive material that produces a specific change in response to a temperature equal to or higher than the predetermined temperature, and a heat conductive material that conducts the heat generated by the living body to the temperature-sensitive material over the predetermined time.

### [Advantageous Effects of Invention]

According to the present invention, it is possible to provide a temperature continuity detector capable of accurately detecting that the detected object has maintained a state of being at a predetermined temperature or at a temperature equal to or higher than the predetermined temperature for a continued period.

### [Brief Description of Drawings]

Fig. 1 shows an example of mounting the temperature continuity detector according to the present invention.
Fig. 2 is a cross-sectional view showing the configuration of the present invention.
Fig. 3 is a cross-sectional view showing the configuration of the present invention.
Fig. 4 is a cross-sectional view showing the configuration of the present invention.
Fig. 5 is a cross-sectional view showing the configuration of the present invention.
Fig. 6 is a cross-sectional view showing the configuration of the present invention.
Fig. 7 is a cross-sectional view showing the configuration of the present invention.
Fig. 8 is a cross-sectional view showing the configuration of the present invention.
Fig. 9 is a cross-sectional view showing the configuration of the present invention.
Fig. 10 is a cross-sectional view showing the configuration of the present invention.
Fig. 11 is a cross-sectional view showing the configuration of the present invention.
Fig. 12 is a cross-sectional view showing the configuration of the present invention.
Fig. 13 is a cross-sectional view showing the configuration of the present invention.
Fig. 14 is a cross-sectional view showing the configuration of the present invention.
Fig. 15 is a cross-sectional view showing the configuration of the present invention.
Fig. 16 is a cross-sectional view showing the configuration of the present invention.
Fig. 17 is a cross-sectional view showing the configuration of the present invention.
Fig. 18 is a cross-sectional view showing the configuration of the present invention.
Fig. 19 is a cross-sectional view showing the configuration of the present invention.
Fig. 20 shows an example of mounting the temperature continuity detector of the present invention.
Fig. 21 shows an example of mounting the temperature continuity detector of the present invention.
Fig. 22 shows an example of the temperature continuity detector of the present invention mounted on another product.
Fig. 23 shows an example of the temperature continuity detector of the present invention mounted on another product.
Fig. 24 shows an example of the temperature continuity detector of the present invention mounted on other parts of the human body.

### [Description of Embodiments]

The contents of the embodiments of the present invention will be listed and described. The present invention includes the following configurations.

### [Item 1]

A temperature continuity detector that detects at least that the detected object, which generates heat, has maintained a predetermined temperature for a predetermined time, or that a temperature equal to or higher than the predetermined temperature has continued for a predetermined time,
wherein the temperature continuity detector comprises a sensor unit having a temperature-sensitive material that reacts to the predetermined temperature to produce a specific change, and a heat conductive material that conducts the heat generated by the detected object to the temperature-sensitive material over the predetermined time.

### [Item 2]

The temperature continuity detector according to item 1,
wherein the heat conductive material is a spacer material having a thickness that allows the heat generated by the detected object to be conducted to the temperature-sensitive material over the predetermined time.

### [Item 3]

The temperature continuity detector according to item 1 or item 2,
wherein the temperature continuity detector further comprises a sub-sensor unit arranged approximately adjacent to the sensor unit, the sub-sensor unit conducting the heat generated by the detected object to the temperature-sensitive material in a time shorter than the predetermined time.

### [Item 4]

The temperature continuity detector according to item 1 or item 2,
wherein the temperature continuity detector further comprises a sub-sensor unit arranged approximately adjacent to the sensor unit, the sub-sensor unit having a sub-spacer material with a thickness smaller than the thickness of the spacer material.

### [Item 5]

The temperature continuity detector according to item 1 or item 2,
wherein the temperature continuity detector further comprises a sub-sensor unit arranged approximately adjacent to the sensor unit, the sub-sensor unit having a temperature-sensitive material that reacts to the predetermined temperature of the detected object to produce a specific change.

### [Item 6]

The temperature continuity detector according to any one of items 3 to 5,
wherein the temperature continuity detector comprises multiple pairs of the sensor units and the sub-sensor units.

### [Item 7]

The temperature continuity detector according to any one of items 1 to 7,
wherein the temperature-sensitive material produces a visible change in response to the predetermined temperature.

### [Item 8]

The temperature continuity detector according to any one of items 1 to 7,
wherein the heat conductive material comprises a material selected from the group consisting of a gas layer, a vacuum layer, foamed resin, resin, wood, cork, polyethylene resin, polyester resin, polypropylene resin, polystyrene resin, acrylic resin, styrene-butadiene resin, styrene-butadiene-acrylonitrile resin, acrylic-styrene resin, ethylene-acrylic resin, styrene-isoprene resin, urethane resin, polyester resin, polycarbonate resin, nylon resin, ethylene-vinyl acetate resin, polyacetal resin, or vinyl chloride resin.

### [Item 9]

The temperature continuity detector according to any one of items 1 to 8,
wherein the sensor unit further comprises a heat conduction equalizing material that contacts the detected object and conducts the heat generated to the heat conductive material substantially uniformly.

### [Item 10]

The temperature continuity detector according to item 9,
wherein the heat conduction equalizing material comprises one material selected from the group consisting of stainless steel, aluminum, platinum, iron, nickel, brass, copper, silver, or an alloy mainly composed of these substances.

### [Item 11]

The temperature continuity detector according to any one of items 1 to 10,
wherein the temperature continuity detector further comprises a heat insulating section that thermally insulates the sensor unit from heat other than the heat generated by the detected object.

### [Item 12]

The temperature continuity detector according to any one of items 1 to 11,
wherein the temperature continuity detector further comprises a fixing section that fixes the sensor unit to a predetermined position of the detected object.

### [Item 13]

A heatstroke detector that detects at least that a living body has maintained a predetermined temperature for a predetermined time, or that a temperature equal to or higher than the predetermined temperature has continued for a predetermined time,
wherein the heatstroke detector comprises a sensor unit having a temperature-sensitive material that reacts to a temperature equal to or higher than the predetermined temperature to produce a specific change, and a heat conductive material that conducts the heat generated by the living body to the temperature-sensitive material over the predetermined time.

### <Detailed Description of Embodiments>

The configuration of a heat stroke detector as an embodiment of the temperature continuity detector according to the present invention will be described below with reference to the drawings. To avoid redundant explanations, identical elements may be given the same or related reference numerals, and detailed descriptions thereof may be omitted.

### <Overview>

The heat stroke detector according to the present invention detects at least one of the following conditions: that the temperature at the attachment site on the body has remained at a predetermined temperature for a predetermined time, or that the temperature has remained above a predetermined temperature for a predetermined time. In other words, the detector detects a state where the temperature at the attachment site has remained within a temperature range at or above the predetermined temperature for the predetermined time (or, alternatively, a state where the temperature at the attachment site has not fallen below the predetermined temperature for the predetermined time).

In general, to detect heat stroke, it is necessary to detect that the core body temperature has remained above a predetermined temperature for a predetermined time. However, it is not practical to continuously insert a temperature sensor into the deep tissues of a living body.

On the other hand, it is not accurate to consider the surface body temperature as equivalent to the core body temperature, as the surface temperature is easily affected by the external environment. Therefore, the heat stroke detector of this embodiment detects that the temperature at the attachment site has remained at a predetermined temperature for a predetermined time. This enables accurate detection of whether the person wearing the heat stroke detector is suffering from heat stroke. Specifically, rather than simply detecting and notifying when the body surface temperature reaches a certain temperature, this heat stroke detector detects when the body surface temperature has reached or exceeded a certain temperature and notifies the user after a predetermined time has elapsed.

To achieve this objective, the heat stroke detector of this embodiment is configured with an intermediate material provided between a contact portion that contacts the skin or other surfaces and a temperature-sensing portion. With this configuration, since the skin temperature (body surface temperature) is transmitted to the temperature-sensing portion through the intermediate material over a predetermined time, when the temperature-sensitive material reacts, it becomes possible to detect that the skin temperature has been at the predetermined temperature for at least the predetermined time.

In conventional technology, notification may occur even when a temperature instantaneously reaches a certain level, thus resulting in detection even in cases where the risk of heat stroke is low.

### <Configuration>

### (First Embodiment)

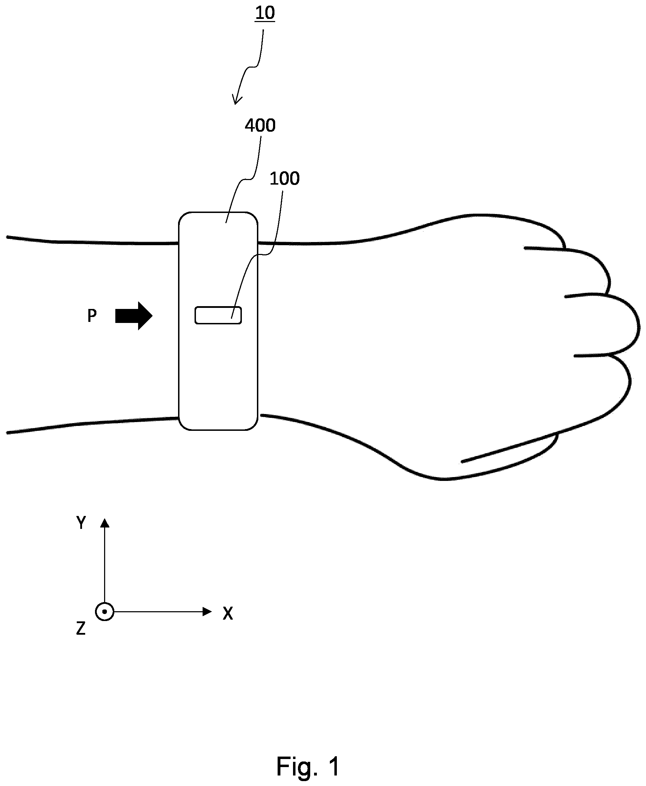

As shown in Figure 1, the heat stroke detector 10 according to this embodiment is configured to be worn on the user's arm. The heat stroke detector 10 includes a sensor unit 100 and is attached to the arm by a fixing portion 400.

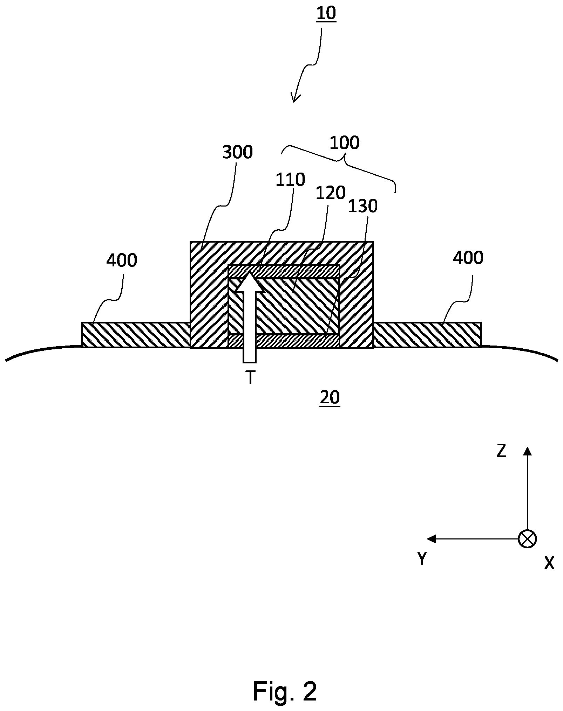

Referring to Figures 1 and 2, the structure of the heat stroke detector 10 will be explained in more detail. Figure 2 is a cross-sectional view of the heat stroke detector 10 shown in Figure 1, viewed from direction P. As shown in Figure 2, the heat stroke detector 10 includes a sensor unit 100, a heat insulating portion 300, and a fixing portion.

The sensor unit 100 includes a temperature-sensitive material 110 that produces a predetermined change in response to a predetermined temperature, a heat conductive material 120 that conducts the temperature of the skin 20 to the temperature-sensitive material over a predetermined time, and a heat conduction equalizing material 130 that contacts the skin 20 and conducts the temperature of the skin 20 substantially uniformly to the heat conductive material.
The temperature-sensitive material 110 produces a visible change at temperatures equal to or higher than a predetermined temperature. As the configuration of the temperature-sensitive material, powders, liquids, or sheets that undergo reversible (or irreversible) color changes in response to temperature changes can be used. For example, the material may be composed of thermochromic ink or synthetic resin containing a thermochromic composition, but is not limited thereto. The material may comprise a leuco dye, a color-developing substance, and a color-change temperature adjusting agent. When the temperature-sensitive material is a powder, it may be mixed with a thermoplastic resin or may be microencapsulated. Furthermore, light-emitting microorganisms capable of bioluminescence may also be utilized.

A visible change by the temperature-sensitive material 110 includes not only changes in hue of the temperature-sensitive material but also changes in brightness or saturation. For example, if the initial color is achromatic, it may continuously change from black to white with increasing temperature. If the initial color has high saturation, it may gradually change from a dark color to a light color as the temperature rises. Additionally, by partially using the temperature-sensitive material, visibility may be achieved through the appearance or disappearance of patterns in response to temperature changes.

The heat conductive material 120 functions as a spacing material having a thickness that allows the temperature of the skin 20 to be conducted to the temperature-sensitive material 110 over a predetermined time. The thickness of the heat conductive material (dimension in the Z-axis direction in the figure) is appropriately selected based on the type of material used and the desired predetermined time (time lag).

The heat conductive material of this embodiment can be selected from various materials, both metallic and non-metallic. Examples include gas layers, vacuum layers, foam resin, resin, wood, and cork, but are not limited thereto. If the thermal conductivity is too high, the aforementioned time lag cannot be effectively created. In other words, it is preferable for the heat conductive material to have a certain degree of thermal resistance. The preferred thermal conductivity of the heat conductive material is preferably 0.01 W/(m·K) or more and 1 W/(m·K) or less, but is not limited thereto.

More specifically, the heat conductive material 120 of this embodiment may employ a thermoplastic resin. The thermoplastic resin may be appropriately selected from, but not limited to, polyethylene resin, polyester resin, polypropylene resin, polystyrene resin, acrylic resin, styrene-butadiene resin, styrene-butadiene-acrylonitrile resin, acrylic-styrene resin, ethylene-acrylic resin, styrene-isoprene resin, urethane resin, polyester resin, polycarbonate resin, nylon resin, ethylene-vinyl acetate resin, polyacetal resin, and vinyl chloride resin.

The heat conductive material is preferably an engineering resin from the viewpoints of processability and thermal conductivity.

The heat conduction equalizing material 130 is provided at a position in contact with the skin 20. The heat conduction equalizing material 130 is provided to conduct the temperature of the skin 20 substantially uniformly to the heat conductive material 120. The heat conduction equalizing material of this embodiment can be selected from various materials, both metallic and non-metallic. However, if the thermal conductivity is too low, non-uniform heat conduction to the heat conductive material will occur, which is undesirable. In other words, it is preferable for the heat conduction equalizing material to have a thermal conductivity above a certain level. The preferred thermal conductivity of the heat conduction equalizing material is preferably 70 W/(m·K) or higher at 25°C, but is not limited thereto.

The heat conduction equalizing material of this embodiment can be appropriately selected from, but is not limited to, stainless steel, aluminum, platinum, iron, nickel, brass, copper, silver, or alloys primarily composed of these materials.

The heat conduction equalizing material is preferably aluminum (foil), particularly from the viewpoints of cost, processability, and corrosion resistance.

The heat insulating portion 300 is provided to thermally insulate the temperature-sensitive material 110, the heat conductive material 120, and the heat conduction equalizing material 130 from heat other than that from the skin 20. As shown in the figure, the heat insulating portion 300 is provided to cover the temperature-sensitive material 110, the heat conductive material 120, and the heat conduction equalizing material 130. This enables the sensor unit 100 to accurately detect only the body temperature.

The heat insulating portion 300 of this embodiment may employ a thermoplastic resin. Additionally, it may comprise a gas layer, vacuum, carbon dioxide, air, helium, or argon. The thermoplastic resin may be appropriately selected from, but not limited to, polyethylene resin, polyester resin, polypropylene resin, polystyrene resin, acrylic resin, styrene-butadiene resin, styrene-butadiene-acrylonitrile resin, acrylic-styrene resin, ethylene-acrylic resin, styrene-isoprene resin, urethane resin, polyester resin, polycarbonate resin, nylon resin, ethylene-vinyl acetate resin, polyacetal resin, and vinyl chloride resin.

From the viewpoint of thermal insulation performance, a vacuum is particularly preferred. Furthermore, the heat insulating portion 300 may be integrally molded with the fixing portion 400 (i.e., the fixing portion 400 may also serve as the heat insulating portion 300), which will be described later. In this case, the material of the heat insulating portion 300 may be the same as that of the fixing portion 400.

The fixing portion 400 fixes the sensor unit 100 and the heat insulating portion 300 at a predetermined position on the user's arm. This maintains the heat conduction equalizing material 130 in contact with the user's skin 20. As mentioned above, a part of the fixing portion may serve as the heat insulating portion 300*

The fixing portion 400 of this embodiment may employ a thermoplastic resin. The thermoplastic resin may be appropriately selected from, but not limited to, rubber resin, silicone resin, and nylon resin.

According to the above configuration, the temperature T of the skin surface reaches the temperature-sensitive material 110 through the heat conduction equalizing material 130 and the heat conductive material 120, as indicated by the arrow in Figure 2. The temperature T is then conducted to the temperature-sensitive material 110 through the heat conductive material 120 over a predetermined time. Therefore, according to this embodiment of the present invention, it is possible to detect that the skin temperature has maintained a predetermined temperature for a predetermined time.

### (Second Embodiment)

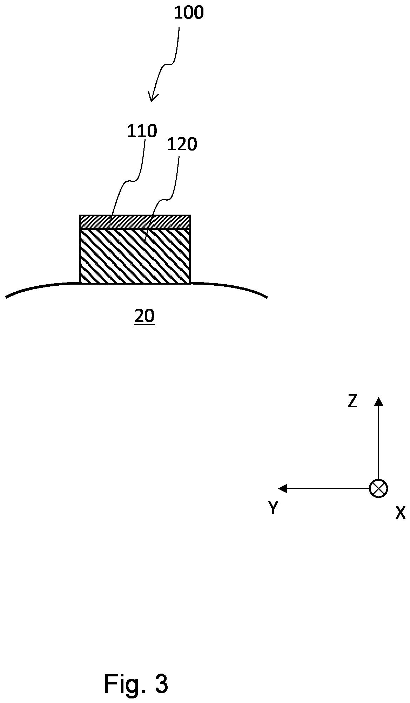

Referring to Figures 3 to 7, a second embodiment of the present invention will be described. The sensor unit 100 of the heat stroke detector according to this embodiment comprises a temperature-sensitive material 110 and a heat conductive material 120.

As shown in Figure 3, the sensor unit 100 is directly attached to the skin 20. The heat stroke detector shown in Figure 3 comprises one sensor unit 100.

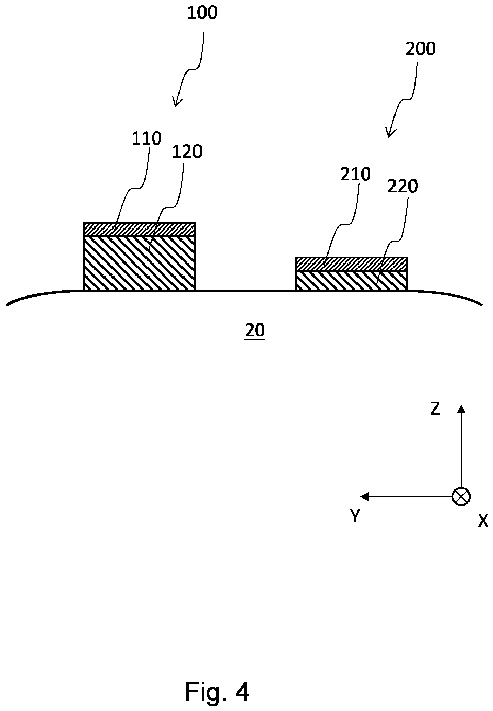

As shown in Figure 4, the heat stroke detector may comprise multiple sensor units. Specifically, the heat stroke detector may comprise a sensor unit 100 and a sub-sensor unit 200. The sensor unit 100 may be similar to the sensor unit shown in Figure 3. The sub-sensor unit 200 comprises a temperature-sensitive material 210 and a heat conductive material 220. The heat conductive material 220 is made of the same material as the heat conductive material 120, but has a different thickness. Consequently, heat from the skin 20 reaches the temperature-sensitive material 210 before reaching the temperature-sensitive material 110. According to this embodiment of the heat stroke detector, since the sub-sensor unit 200 can detect before the sensor unit 100, the sub-sensor unit 200 can function as an early warning indicator. By monitoring changes in the color or other characteristics of the sub-sensor unit 200, the user can recognize early signs of potential heat stroke, thus enabling safer activities. The thickness and material of the heat conductive material 220 can be adjusted according to the desired timing of the early warning.

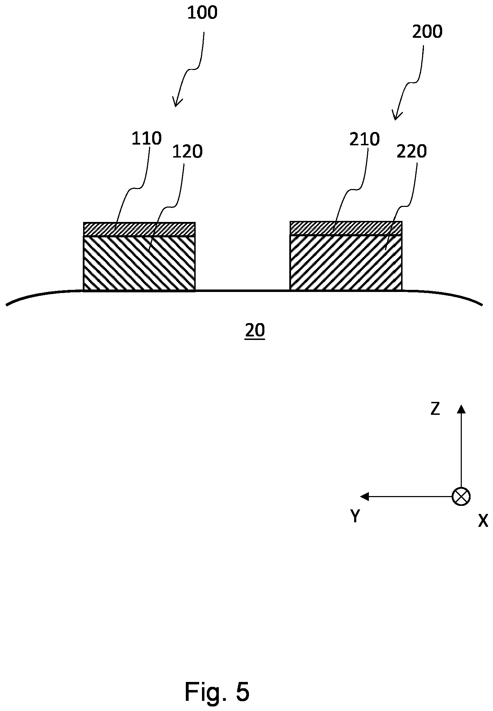

As shown in Figure 5, the heat conductive material 220 may have the same thickness as the heat conductive material 120 provided it has a different thermal conductivity. In other words, the different thermal conductivity should be sufficient to enable the early warning function described above. Following the same principle, the heat conductive material 220 may have both a different thermal conductivity and a different thickness from the heat conductive material 120.

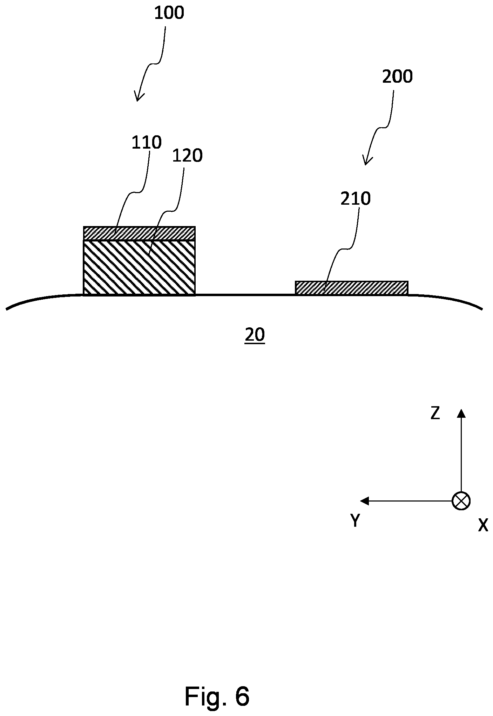

The heat stroke detector shown in Figure 6 also comprises multiple sensor units. However, the structure of the sub-sensor unit 200 is different. The sub-sensor unit 200 comprises only a temperature-sensitive material 210. This configuration enables earlier early warning detection of heat stroke simply by reducing the number of components.

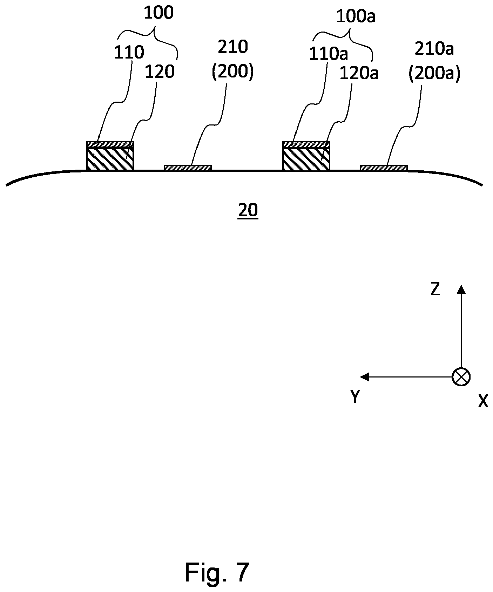

The heat stroke detector shown in Figure 7 comprises two pairs of sensor unit 100 and sub-sensor unit 200. The structures of the sensor units 100 and 100a and the sub-sensor units 200 and 200a are similar to those shown in Figure 5; however, the reaction temperatures of the temperature-sensitive materials 110a and 210a are different from those of the temperature-sensitive materials 110 and 210. Thus, by providing multiple pairs of sensor units and sub-sensor units, the pair that reacts first can serve as an early warning function.

### (Third Embodiment)

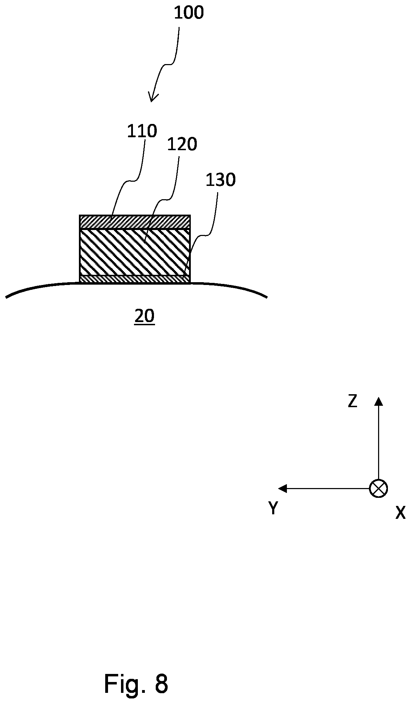

Referring to Figures 8 to 11, a third embodiment of the present invention will be described. The sensor unit 100 of the heat stroke detector according to this embodiment comprises a temperature-sensitive material 110, a heat conductive material 120, and a heat conduction equalizing material 130.

As shown in Figure 8, the sensor unit 100 is directly attached to the skin 20. The heat conduction equalizing material 130 is in contact with the skin 20. The heat stroke detector shown in Figure 8 comprises one sensor unit 100.

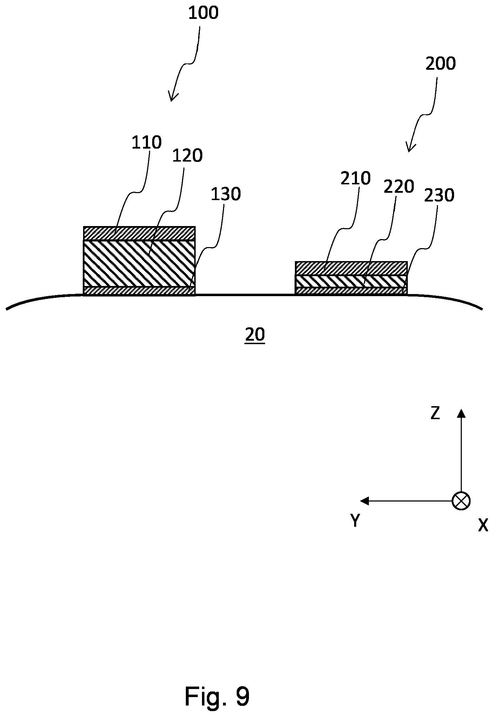

As shown in Figure 9, the heat stroke detector may comprise multiple sensor units. Specifically, the heat stroke detector may comprise a sensor unit 100 and a sub-sensor unit 200. The sensor unit 100 may be similar to the sensor unit shown in Figure 8. The sub-sensor unit 200 comprises a temperature-sensitive material 210, a heat conductive material 220, and a heat conduction equalizing material 230. The heat conductive material 220 is made of the same material as the heat conductive material 120, but has a different thickness. As described above, the heat conductive material 220 may have the same thickness as the heat conductive material 120 provided it has a different thermal conductivity, or it may have both a different thermal conductivity and a different thickness from the heat conductive material 120. Consequently, heat from the skin 20 reaches the temperature-sensitive material 210 before reaching the temperature-sensitive material 110. According to this embodiment of the heat stroke detector, since the sub-sensor unit 200 can detect before the sensor unit 100, the sub-sensor unit 200 can function as an early warning indicator. By monitoring changes in the color or other characteristics of the sub-sensor unit 200, the user can recognize early signs of potential heat stroke, thus enabling safer activities. The thickness and material of the heat conductive material 220 can be adjusted according to the desired timing of the early warning.

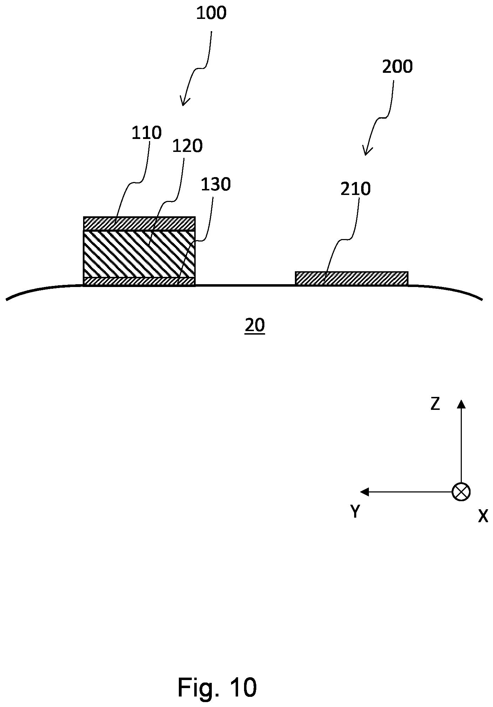

The heat stroke detector shown in Figure 10 also comprises multiple sensor units. However, the structure of the sub-sensor unit 200 is different. The sub-sensor unit 200 comprises only a temperature-sensitive material 210. This configuration enables earlier early warning detection of heat stroke simply by reducing the number of components.

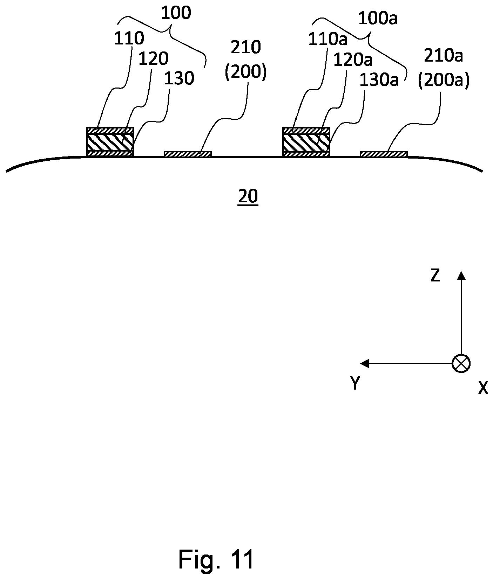

The heat stroke detector shown in Figure 11 comprises two pairs of sensor unit 100 and sub-sensor unit 200. The structures of the sensor units 100 and 100a and the sub-sensor units 200 and 200a are similar to those shown in Figure 9; however, the reaction temperatures of the temperature-sensitive materials 110a and 210a are different from those of the temperature-sensitive materials 110 and 210. Thus, by providing multiple pairs of sensor units and sub-sensor units, the pair that responds earlier can serve as an early warning function.

### (Fourth Embodiment)

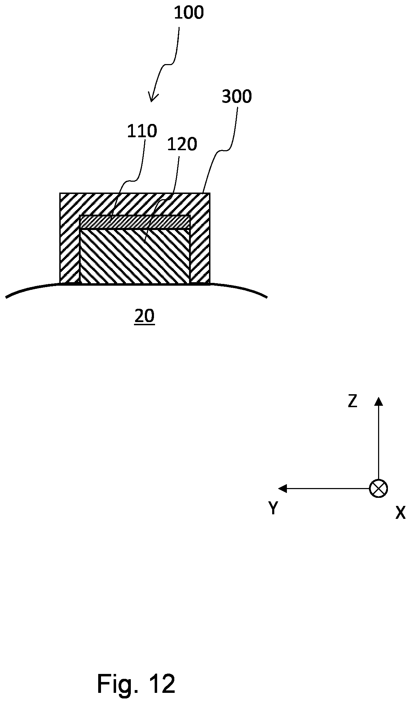

Referring to Figures 12 to 15, a fourth embodiment of the present invention will be described. The sensor unit 100 of the heat stroke detector according to this embodiment comprises a temperature-sensitive material 110, a heat conductive material 120, and a heat insulating portion 300.

As shown in Figure 12, the sensor unit 100 is directly attached to the skin 20. The heat conductive material 120 is in contact with the skin 20. The heat stroke detector comprises one sensor unit 100.

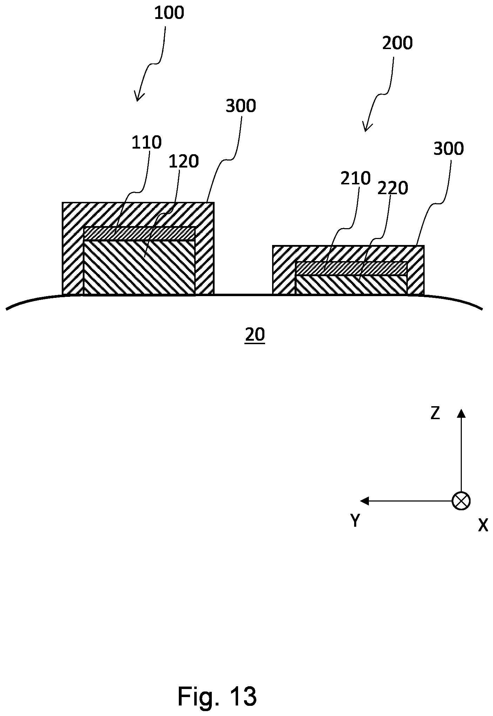

As shown in Figure 13, the heat stroke detector may comprise multiple sensor units. Specifically, the heat stroke detector may comprise a sensor unit 100 and a sub-sensor unit 200. The sensor unit 100 may be similar to the sensor unit shown in Figure 12. The sub-sensor unit 200 comprises a temperature-sensitive material 210, a heat conductive material 220, and a heat insulating portion 300. The heat conductive material 220 is made of the same material as the heat conductive material 120, but has a different thickness. As described above, the heat conductive material 220 may have the same thickness as the heat conductive material 120 provided it has a different thermal conductivity, or it may have both a different thermal conductivity and a different thickness from the heat conductive material 120. Consequently, heat from the skin 20 reaches the temperature-sensitive material 210 before reaching the temperature-sensitive material 110. According to this embodiment of the heat stroke detector, since the sub-sensor unit 200 can detect before the sensor unit 100, the sub-sensor unit 200 can function as an early warning indicator. By monitoring changes in the color or other characteristics of the sub-sensor unit 200, the user can recognize early signs of potential heat stroke, thus enabling safer activities. The thickness and material of the heat conductive material 220 can be adjusted according to the desired timing of the early warning.

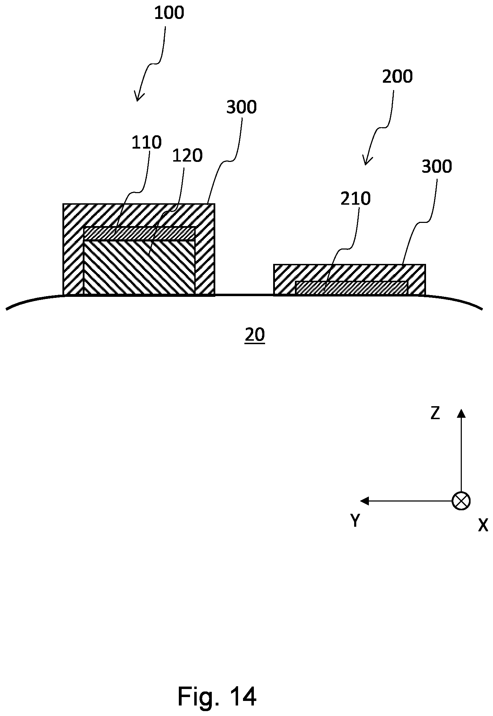

The heat stroke detector shown in Figure 14 also comprises multiple sensor units. However, the structure of the sub-sensor unit 200 is different. The sub-sensor unit 200 comprises only a temperature-sensitive material 210 and a heat insulating portion 300. This configuration enables earlier early warning detection of heat stroke simply by reducing the number of components.

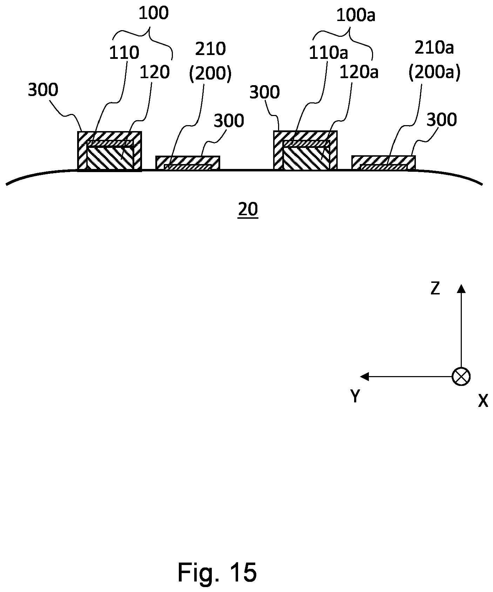

The heat stroke detector shown in Figure 15 comprises two pairs of sensor unit 100 and sub-sensor unit 200. The structures of the sensor units 100 and 100a and the sub-sensor units 200 and 200a are similar to those shown in Figure 14; however, the reaction temperatures of the temperature-sensitive materials 110a and 210a are different from those of the temperature-sensitive materials 110 and 210. Thus, by providing multiple pairs of sensor units and sub-sensor units, the pair that responds earlier can serve as an early warning function.

### (Fifth Embodiment)

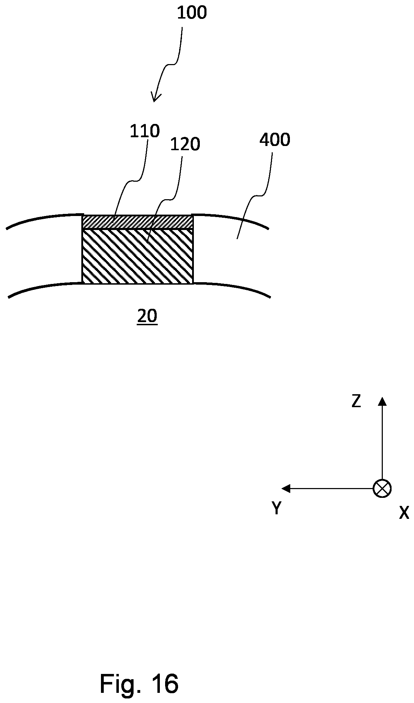

Referring to Figures 16 to 19, a fifth embodiment of the present invention will be described. The sensor unit 100 of the heat stroke detector according to this embodiment comprises a temperature-sensitive material 110, a heat conductive material 120, and a fixing portion 400.

As shown in Figure 16, the sensor unit 100 is directly attached to the skin 20. The fixing portion 400 fixes the sensor unit 100 to the user's arm or other body parts. The heat stroke detector comprises one sensor unit 100.

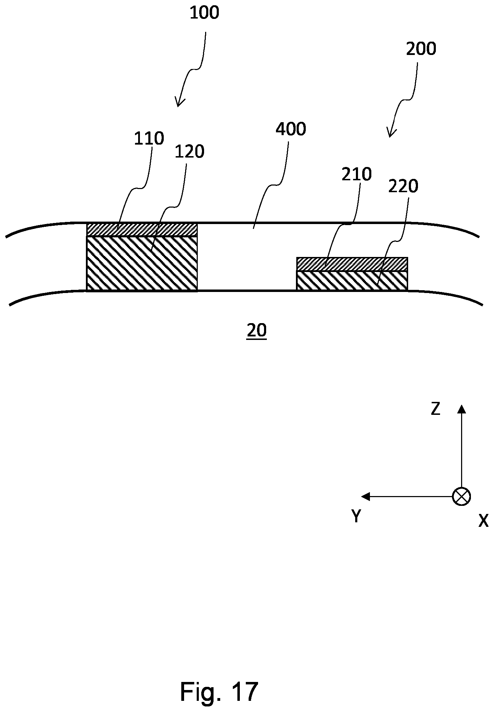

As shown in Figure 17, the heat stroke detector may comprise multiple sensor units. Specifically, the heat stroke detector may comprise a sensor unit 100 and a sub-sensor unit 200. These sensor units are formed to be contained within the fixing portion 400. The sensor unit 100 may be similar to the sensor unit shown in Figure 16. The sub-sensor unit 200 comprises a temperature-sensitive material 210 and a heat conductive material 220. The heat conductive material 220 is made of the same material as the heat conductive material 120, but has a different thickness. As described above, the heat conductive material 220 may have the same thickness as the heat conductive material 120 provided it has a different thermal conductivity, or it may have both a different thermal conductivity and a different thickness from the heat conductive material 120. Consequently, heat from the skin 20 reaches the temperature-sensitive material 210 before reaching the temperature-sensitive material 110. According to this embodiment of the heat stroke detector, since the sub-sensor unit 200 can detect before the sensor unit 100, the sub-sensor unit 200 can function as an early warning indicator. By monitoring changes in the color or other characteristics of the sub-sensor unit 200, the user can recognize early signs of potential heat stroke, thus enabling safer activities. The thickness and material of the heat conductive material 220 can be adjusted according to the desired timing of the early warning.

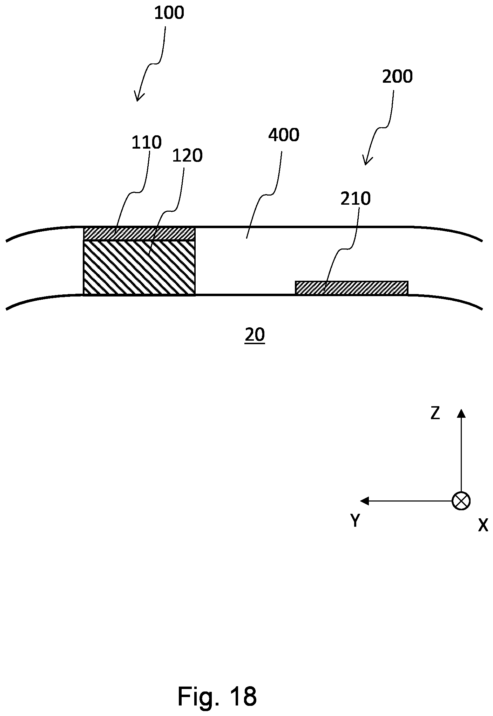

The heat stroke detector shown in Figure 18 also comprises multiple sensor units. However, the structure of the sub-sensor unit 200 is different. The sub-sensor unit 200 comprises only a temperature-sensitive material 210. This configuration enables earlier early warning detection of heat stroke simply by reducing the number of components.

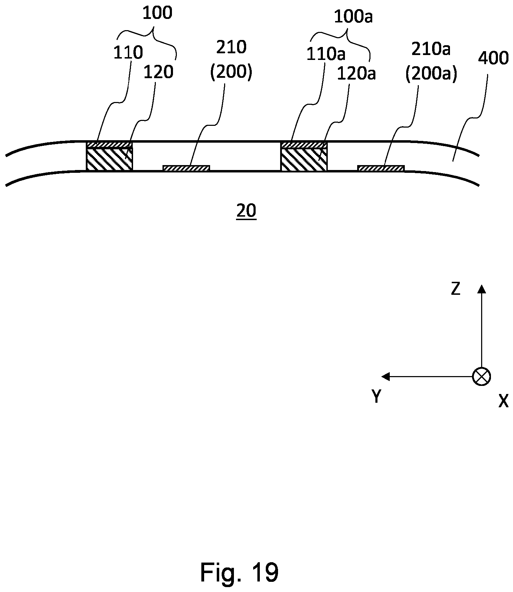

The heat stroke detector shown in Figure 19 comprises two pairs of sensor unit 100 and sub-sensor unit 200. The structures of the sensor units 100 and 100a and the sub-sensor units 200 and 200a are similar to those shown in Figure 17; however, the reaction temperatures of the temperature-sensitive materials 110a and 210a are different from those of the temperature-sensitive materials 110 and 210. Thus, by providing multiple pairs of sensor units and sub-sensor units, the pair that responds earlier can serve as an early warning function.

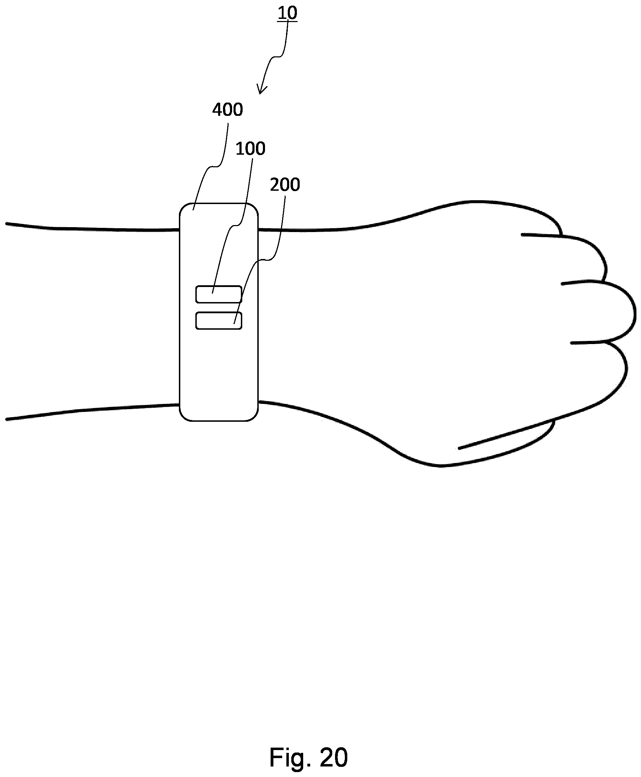

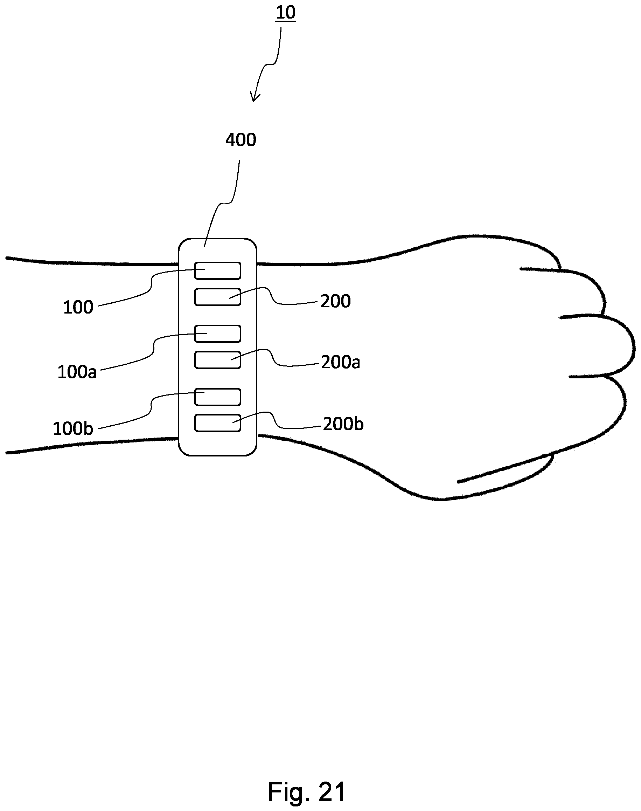

As shown in Figure 20, both the sensor unit 100 and sub-sensor unit 200 are visibly provided in the fixing portion 400. As shown in Figure 21, three (or more) sets of sensor units 100, 100a, 100b and sub-sensor units 200a, 200b, 200c may be provided.

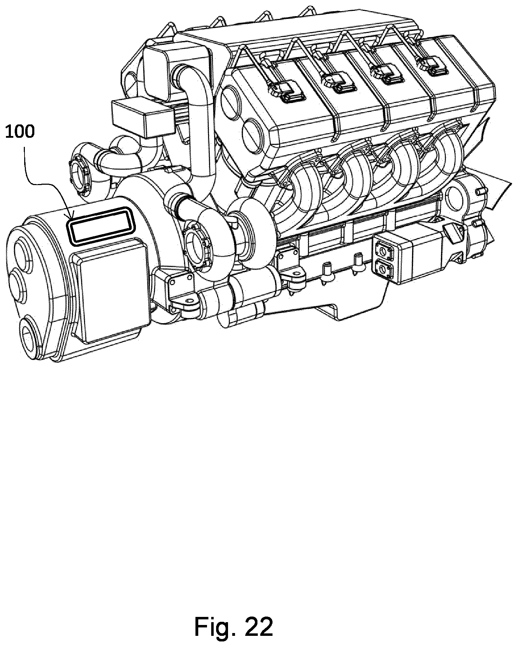

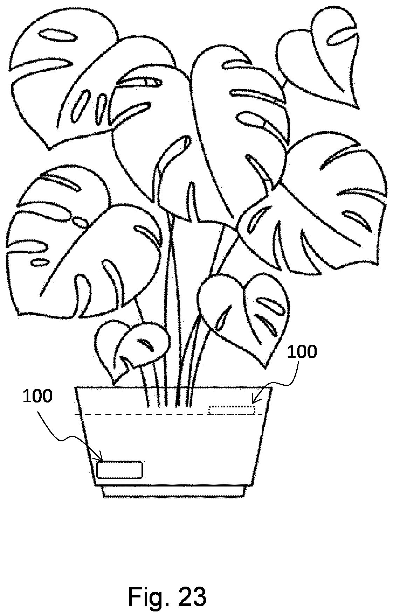

The heat stroke detector according to the embodiments described above is an application of the temperature continuity detector to the human body; however, the industrial applications of the present invention are not limited thereto. For example, as shown in Figure 22, the sensor unit 100 may be attached to a device for detecting whether it has been operating at a predetermined temperature for a predetermined time (such as for detecting thermal runaway). Additionally, as shown in Figure 23, it may be used to monitor whether plants or other objects have exceeded a predetermined temperature. In such cases, the sensor unit may be directly placed on a flowerpot or in soil.

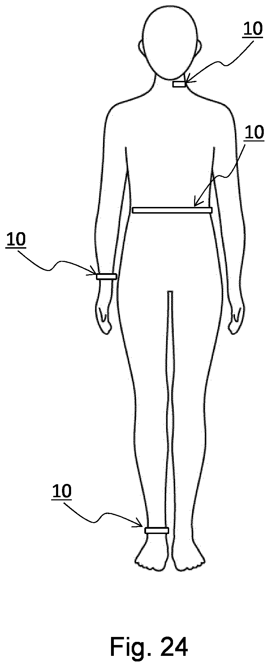

As shown in Figure 24, it can also be applied to other parts of the human body. For example, the temperature continuity detector 10 may be attached not only to the arm but also to the ankle, torso, or neck surface for detecting heat stroke or similar conditions.

In the above-described embodiments of the present invention, various configurations of the sensor unit (and the sub-sensor unit) have been described, comprising combinations of a temperature-sensitive material, a heat conductive material, a heat conduction equalizing material, a heat insulating portion, and a fixing portion. However, the present invention is not limited to these combinations. For example, the sensor unit may comprise:
only a temperature-sensitive material and a heat conductive material (Configuration 1-1); or
a temperature-sensitive material, a heat conductive material, and a heat conduction equalizing material (Configuration 1-2).

The sub-sensor unit may comprise:
only a temperature-sensitive material (Configuration 2-1);
a temperature-sensitive material and a heat conductive material (Configuration 2-2); or
a temperature-sensitive material, a heat conductive material, and a heat conduction equalizing material (Configuration 2-3).

The detector may comprise either a single sensor unit or a combination of sensor units and sub-sensor units (in various quantities). Furthermore, it may include a heat insulating portion, a fixing portion, or a combination of both.

The above-described embodiments are merely examples to facilitate understanding of the invention and are not intended to be construed as limiting the invention. It goes without saying that the invention may be changed and improved without departing from its purpose, and that the invention includes its equivalents.

### [Reference Signs List]

- 10: Temperature continuity detector
- 20: Skin (detection object)
- 100, 100a, 100b: Sensor unit
- 110, 110a: Temperature-sensitive material
- 120, 120a: Heat conductive material (spacing material)
- 130: Heat conduction equalizing material
- 200, 200a, 200b: Sub-sensor unit
- 210, 210a: Temperature-sensitive material
- 220: Heat conductive material (sub-spacing material)
- 230: Heat conduction equalizing material
- 300: Heat insulating portion
- 400: Fixing portion

## Claims

1. A temperature continuity detector that detects at least that the detected object, which generates heat, has maintained a predetermined temperature for a predetermined time, or that a temperature equal to or higher than the predetermined temperature has continued for a predetermined time,
wherein the temperature continuity detector comprises a sensor unit having a temperature-sensitive material that reacts to the predetermined temperature to produce a specific change, and a heat conductive material that conducts the heat generated by the detected object to the temperature-sensitive material over the predetermined time.

2. The temperature continuity detector according to claim 1,
wherein the heat conductive material is a spacer material having a thickness that allows the heat generated by the detected object to be conducted to the temperature-sensitive material over the predetermined time.

3. The temperature continuity detector according to claim 1,
wherein the temperature continuity detector further comprises a sub-sensor unit arranged approximately adjacent to the sensor unit, the sub-sensor unit conducting the heat generated by the detected object to the temperature-sensitive material in a time shorter than the predetermined time.

4. The temperature continuity detector according to claim 3,
wherein the temperature continuity detector further comprises a sub-sensor unit arranged approximately adjacent to the sensor unit, the sub-sensor unit having a sub-spacer material with a thickness smaller than the thickness of the spacer material.

5. The temperature continuity detector according to claim 3,
wherein the temperature continuity detector further comprises a sub-sensor unit arranged approximately adjacent to the sensor unit, the sub-sensor unit having a temperature-sensitive material that reacts to the predetermined temperature of the detected object to produce a specific change.

6. The temperature continuity detector according to claim 3,
wherein the temperature continuity detector comprises multiple pairs of the sensor units and the sub-sensor units.

7. The temperature continuity detector according to claim 1,
wherein the temperature-sensitive material produces a visible change in response to the predetermined temperature.

8. The temperature continuity detector according to claim 1,
wherein the heat conductive material comprises a material selected from the group consisting of a gas layer, a vacuum layer, foamed resin, resin, wood, cork, polyethylene resin, polyester resin, polypropylene resin, polystyrene resin, acrylic resin, styrene-butadiene resin, styrene-butadiene-acrylonitrile resin, acrylic-styrene resin, ethylene-acrylic resin, styrene-isoprene resin, urethane resin, polyester resin, polycarbonate resin, nylon resin, ethylene-vinyl acetate resin, polyacetal resin, or vinyl chloride resin.

9. The temperature continuity detector according to claim 1,
wherein the sensor unit further comprises a heat conduction equalizing material that contacts the detected object and conducts the heat generated to the heat conductive material substantially uniformly.

10. The temperature continuity detector according to claim 9,
wherein the heat conduction equalizing material comprises one material selected from the group consisting of stainless steel, aluminum, platinum, iron, nickel, brass, copper, silver, or an alloy mainly composed of these substances.

11. The temperature continuity detector according to claim 1,
wherein the temperature continuity detector further comprises a heat insulating section that thermally insulates the sensor unit from heat other than the heat generated by the detected object.

12. The temperature continuity detector according to claim 1,
wherein the temperature continuity detector further comprises a fixing section that fixes the sensor unit to a predetermined position of the detected object.

13. A heatstroke detector that detects at least that a living body has maintained a predetermined temperature for a predetermined time, or that a temperature equal to or higher than the predetermined temperature has continued for a predetermined time,
wherein the heatstroke detector comprises a sensor unit having a temperature-sensitive material that reacts to a temperature equal to or higher than the predetermined temperature to produce a specific change, and a heat conductive material that conducts the heat generated by the living body to the temperature-sensitive material over the predetermined time.
